(19) Europäisches Patentamt
European Patent Office
Office européen des brevets

(11) **EP 3 722 439 B1**

(12) **FASCICULE DE BREVET EUROPEEN**

(45) Date de publication et mention
de la délivrance du brevet:
**10.05.2023 Bulletin 2023/19**

(21) Numéro de dépôt: **20169054.2**

(22) Date de dépôt: **09.04.2020**

(51) Classification Internationale des Brevets (IPC):
*C12Q 1/06* *(2006.01)*     *G01N 15/06* *(2006.01)*
*G01N 15/14* *(2006.01)*     *G03H 1/04* *(2006.01)*
*G06V 10/44* *(2022.01)*     *G06V 10/764* *(2022.01)*
*G06V 10/82* *(2022.01)*     *G06V 20/69* *(2022.01)*

(52) Classification Coopérative des Brevets (CPC):
**C12Q 1/06; G01N 15/06; G01N 15/1434;
G01N 15/1463; G06V 10/454; G06V 10/764;
G06V 10/82; G06V 20/69;** G01N 2015/0065;
G01N 2015/0687; G01N 2015/0693;
G01N 2015/1006; G01N 2015/144;
G01N 2015/1454; G03H 1/0443;    (Cont.)

(54) **PROCÉDÉ D'OBSERVATION PRÉCOCE DE COLONIES DE MICROORGANISMES**

FRÜHZEITIGES BEOBACHTUNGSVERFAHREN VON MIKROORGANISMUS-KOLONIEN

METHOD FOR EARLY OBSERVATION OF COLONIES OF MICROORGANISMS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **12.04.2019 FR 1903964**

(43) Date de publication de la demande:
**14.10.2020 Bulletin 2020/42**

(73) Titulaire: **Commissariat à l'énergie atomique
et aux énergies alternatives
75015 Paris (FR)**

(72) Inventeurs:
• **BORDY, Thomas**
**38054 GRENOBLE Cedex 09 (FR)**
• **ALLIER, Cédric**
**38054 GRENOBLE Cedex 09 (FR)**
• **MARCOUX, Pierre**
**38054 GRENOBLE Cedex 09 (FR)**

(74) Mandataire: **INNOV-GROUP**
**310, avenue Berthelot**
**69372 Lyon Cedex 08 (FR)**

(56) Documents cités:
**EP-A1- 3 270 232**     **WO-A1-2018/215337**
**US-A1- 2018 285 624**

• **YOSHIAKI MAEDA ET AL: "Colony fingerprint for
discrimination of microbial species based on
lensless imaging of microcolonies", PLOS ONE,
vol. 12, no. 4, 3 avril 2017 (2017-04-03), page
e0174723, XP055660462, DOI:
10.1371/journal.pone.0174723**

**(Cont. page suivante)**

(52) Classification Coopérative des Brevets (CPC):
(Cont.)
G03H 2001/005; G03H 2001/0447; G03H 2001/045

## Description

### DOMAINE TECHNIQUE

Le domaine technique de l'invention est l'observation de colonies de microorganismes, et en particulier des colonies se développant dans un milieu de culture non transparent.

### ART ANTERIEUR

L'observation de colonies bactériennes par imagerie est une technique connue depuis bien longtemps dans le domaine de la microbiologie, pour effectuer un suivi du développement de de microorganismes ou de cellules. Dans une boîte de Pétri, il est possible de suivre le développement de colonies, par exemple de colonies bactériennes, et de les dénombrer. La forme des colonies donne une information sur le type de microorganisme. En outre, en combinant l'utilisation de différents milieux de culture, permettant ou ne permettant pas le développement de colonies, il est possible d'identifier le type de microorganisme formant des colonies.

La caractérisation de microorganismes sur des boîtes de Pétri est encore une méthode de référence, dont l'utilisation est fréquente dans le domaine de la microbiologie, du diagnostic, mais également dans le domaine de l'agroalimentaire ou de la cosmétique. Le principal inconvénient de cette méthode est sa lenteur, puisqu'il faut généralement attendre plusieurs journées pour obtenir un résultat exploitable. Un autre inconvénient est que cette méthode est difficilement automatisable, et requiert des opérateurs humains expérimentés.

Récemment, des méthodes holographiques ont permis le dénombrement et la caractérisation de microorganismes, et constituent des alternatives prometteuses aux techniques existantes, en permettant une meilleure automatisation. Par exemple, le brevet US7465560 décrit une méthode permettant de caractériser un microorganisme basée sur l'exploitation de la diffusion et de la diffraction, par le microorganisme, d'un faisceau laser incident. Le microorganisme est disposé entre une source de lumière laser et un capteur d'image. Sous l'effet d'une illumination par le faisceau laser, on acquiert une image sur laquelle des figures de diffractions apparaissent, ces dernières constituant une signature du microorganisme observé. Le brevet US8787633 décrit une méthode répondant au même objectif.

Par ailleurs, la publication Meada Y "Colony fingerprint for discrimination of microbial species based on lensless imaging of microcolonies", PLoS ONE 12(4) (2017), décrit un procédé d'observation de colonies bactériennes selon une configuration d'imagerie sans lentille. Les colonies se développent dans une gélose LB Agar (Lysogeny Broth Agar) d'épaisseur 600 $\mu$m. Cette gélose est transparente, ce qui permet une observation correcte des colonies.

Cependant, les méthodes holographiques précédemment décrites deviennent inapplicables dès lors que le milieu, dans lequel sont disposés les microorganismes, est opaque, coloré ou diffusant. En effet, ces méthodes utilisent une image formée selon une configuration dite en transmission, dans laquelle l'échantillon est disposé entre une source de lumière et un capteur d'image. L'obtention d'une image exploitable est soumise à l'utilisation d'un échantillon suffisamment transparent. Ainsi, cette méthode n'est pas compatible avec des échantillons comportant un milieu de culture coloré et/ou diffusant, par exemple le milieu connu sous la désignation Columbia Blood Sheep comportant une gélose Columbia au sang de mouton. Elle n'est également pas applicable à un milieu opaque et diffusant de type gélose chocolat. Or, de tels milieux de culture sont fréquemment utilisés dans le diagnostic clinique.

Les documents WO2018122504 et WO2018122505 et décrivent une méthode d'identification bactérienne selon une configuration en rétrodiffusion : une source de lumière illumine une colonie et un capteur d'image acquiert une image à partir d'un rayonnement réfléchi par la colonie bactérienne. Cette méthode est bien adaptée à l'identification de colonies, mais elle ne permet pas de dénombrer efficacement des colonies réparties dans un échantillon, sauf à effectuer un balayage de l'échantillon.

Le document WO2018215337 décrit un procédé d'identification de microorganismes vivant à partir d'une image acquise selon une configuration sans lentille. Les microorganismes font l'objet d'un marquage à l'aide d'un indicateur de viabilité, qui modifie la couleur des microorganismes selon qu'il sont morts ou vivants. Le procédé implique le recours à un algorithme de propagation holographique, appliqué à l'image acquise, de façon à former une image exploitable des microorganismes. Le document FR 3054037 décrit également un dispositif d'observation d'un échantillon à partir d'une image acquise selon une configuration sans lentille.

Les inventeurs ont conçu un procédé optique de numération de colonies de microorganismes particulièrement rapide, permettant une caractérisation précoce d'échantillons. Le procédé peut être appliqué sans nécessiter un marquage des colonies.

### EXPOSE DE L'INVENTION

Un objet de l'invention est un procédé d'observation d'un échantillon selon la revendication 1. Le milieu de culture peut s'étendre, parallèlement à l'axe de propagation, selon une épaisseur inférieure à 250 $\mu$m ou à 100 $\mu$m.

Le milieu de culture s'étend entre une face supérieure et une face inférieure, perpendiculaires ou perpendiculaires à 20° près, à l'axe de propagation. Sous l'effet de leur développement, les microorganismes forment des colonies, au moins une colonie formant un canal de lumière s'étendant de la face supérieure jusqu'à la face inférieure, à travers le milieu de culture, de telle sorte qu'au moins une colonie forme une tache lumineuse sur l'image acquise par le capteur d'image. Selon ce mode de réalisation, le procédé peut comporter:

- un comptage de taches lumineuses formées sur l'image acquise par le capteur d'image;
- une estimation du nombre de colonies dans l'échantillon, en fonction du nombre de taches lumineuses comptées sur l'image.

Par tache lumineuse, il est entendu une zone ponctuelle, regroupant par exemple quelques dizaines ou quelques centaines de pixels, dont l'intensité est supérieure aux pixels adjacents de la zone ponctuelle.
Le procédé peut comporter :

- une analyse morphologique d'au moins une tache lumineuse formée sur l'image acquise par le capteur d'image;
- une identification de la colonie, ayant engendré la tache lumineuse, à partir de l'analyse morphologique.

Par identification d'une colonie, on entend une détermination de l'espèce des microorganismes formant la colonie.
Selon un mode de réalisation non revendiqué, l'image acquise comporte au moins une figure de diffraction, associée à une colonie de microorganismes. Le procédé comporte alors :

- un comptage de chaque figure de diffraction, de façon à estimer une quantité de colonies dans l'échantillon ;
- et/ou une analyse morphologique d'au moins une figure de diffraction, ainsi qu'une identification de la colonie, associée à la figure de diffraction, à partir de l'analyse morphologique.

Selon un mode de réalisation non revendiqué, le procédé comporte :

- une mise en oeuvre d'un réseau de neurones, à partir de l'image acquise, pour détecter les figures de diffraction ;
- un comptage des figures de diffraction ainsi détectées.

Le réseau de neurones peut avoir été préalablement paramétré par une phase d'apprentissage, en utilisant des échantillons comportant des microorganismes dont la position est connue, et dont l'espèce est de préférence connue.
Selon un mode de réalisation non revendiqué, le procédé comporte :

- une acquisition d'une première image, à un premier instant, l'image comportant au moins une figure de diffraction associée à une colonie de microorganismes ;
- une acquisition d'une deuxième image, à un deuxième instant, postérieur au premier instant, la deuxième image comportant au moins une tache lumineuse, associée à la colonie de microorganismes.

Selon un mode de réalisation :

- le milieu de culture s'étend dans une chambre de confinement;
- le milieu de culture s'étend entre deux faces opposées, perpendiculaires ou sensiblement perpendiculaires à l'axe de propagation ;
- la chambre de confinement est au contact du milieu de culture au niveau des deux faces opposées.

Selon un mode de réalisation, aucune optique de formation d'image n'est disposée entre l'échantillon et le capteur d'image.
Selon un mode de réalisation,

- un système optique de formation d'image est disposé entre l'échantillon et le capteur d'image, le système optique définissant un plan objet et un plan image;
- le capteur d'image définit un plan de détection ; le procédé étant tel que, lors de l'acquisition de l'image :
- l'échantillon est décalé par rapport au plan objet selon une distance de défocalisation objet ;
- et/ou le plan de détection est décalé, par rapport au plan image, selon une distance de défocalisation image.

Par système optique de formation d'image, on entend un objectif ou une lentille.
Selon un mode de réalisation,

- un système optique de formation d'image est disposé entre l'échantillon et le capteur d'image, le système optique définissant un plan objet et un plan image;
- le capteur d'image définit un plan de détection ;

le procédé étant tel que, lors de l'acquisition de l'image la face inférieure de l'échantillon correspond au plan objet et le plan de détection correspond au plan image.
L'invention sera mieux comprise à la lecture de l'exposé des exemples de réalisation présentés, dans la suite de la description, en lien avec les figures listées ci-dessous.

## FIGURES

Les figures 1A et 1B représentent un exemple de dispositif permettant une mise en oeuvre de l'invention, selon une configuration d'imagerie sans lentille. La figure 1C représente un dispositif de l'invention dans lequel un système optique s'étend entre l'échantillon et le capteur d'image.

La figure 2 montre des parties d'images d'un échantillon acquises à différents instants après un instant initial.

Les figures 3A et 3B montrent des images d'un échantillon respectivement acquises à un instant initial et 4 heures après l'instant initial.

La figure 4 montre une évolution de l'atténuation optique d'une gélose en fonction de son épaisseur.

La figure 5A schématise une architecture d'un réseau de neurones convolutif.

La figure 5B est une vignette d'entrée utilisée lors de l'apprentissage du réseau de neurones.

La figure 5C est une vignette de sortie utilisée lors de l'apprentissage du réseau de neurones.

La figure 6A est une image comportant des figures de diffraction.

La figure 6B représente une détection de figures de diffraction à partir de l'image de la figure 6A.

**EXPOSE DE MODES DE REALISATION PARTICULIERS**

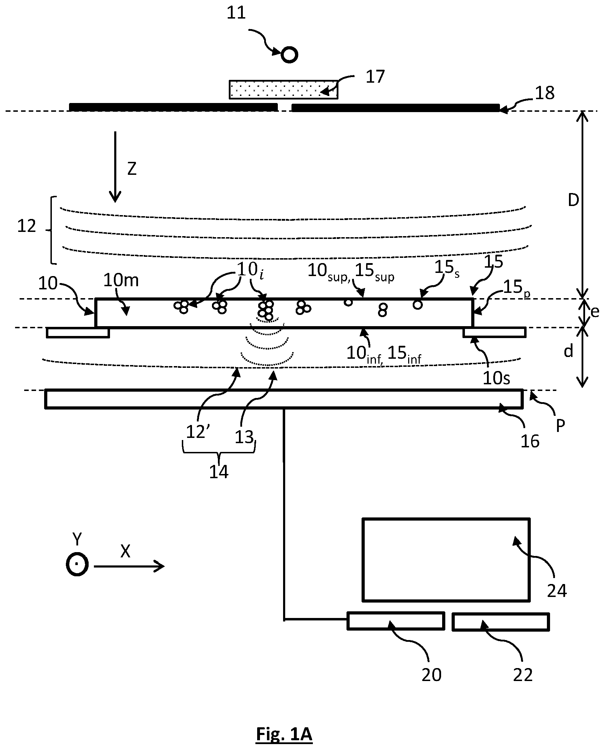

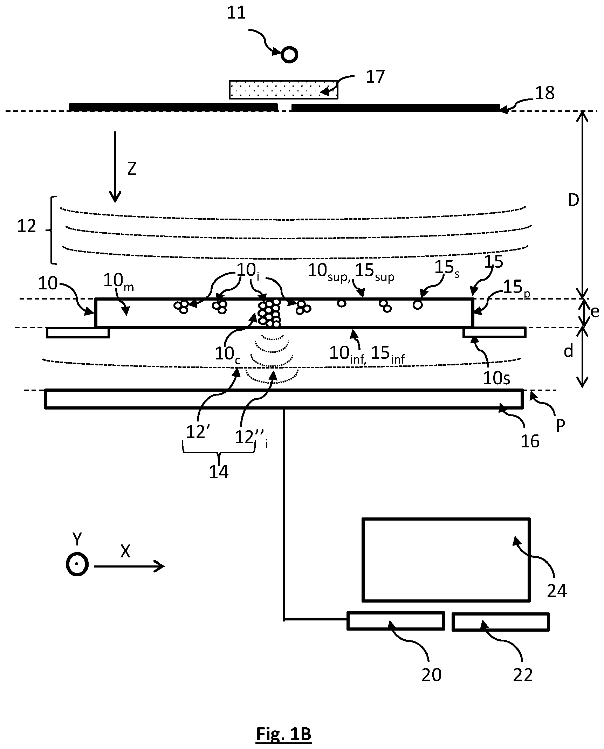

Les figures 1A et 1B représentent un exemple de dispositif permettant une application d'un procédé selon l'invention. Une source de lumière 11 est configurée pour émettre une onde lumineuse 12, dite onde lumineuse incidente, se propageant en direction d'un échantillon 10, selon un axe de propagation Z. L'onde lumineuse est émise selon une bande spectrale d'illumination $\Delta\lambda$.

L'échantillon 10 comporte des microorganismes 10j que l'on souhaite détecter, afin de les dénombrer ou de les caractériser, par exemple en les identifiant. Les microorganismes baignent dans un milieu $10_m$, formant un milieu de culture, propice au développement des microorganismes, et en particulier à des colonies de microorganismes. Le milieu $10_m$ comporte des nutriments permettant le développement des microorganismes. Par microorganisme, on entend notamment une levure, une bactérie, une spore, un champignon ou une cellule, qu'il s'agisse d'une cellule eucaryote ou procaryote, ou une microalgue.

Lorsque les microorganismes forment des colonies, la concentration peut par exemple être inférieure à 1000 colonies de microorganismes par mm². L'échantillon est confiné dans une chambre de confinement 15. Cette dernière est maintenue, entre la source de lumière 11 et un capteur d'image 16, par un support d'échantillon 10s.

La distance D entre la source de lumière 11 et la chambre de confinement 15 est de préférence supérieure à 1 cm. Elle est de préférence comprise entre 2 et 30 cm. Avantageusement, la source de lumière, vue par l'échantillon, est considérée comme ponctuelle. Cela signifie que son diamètre (ou sa diagonale) est préférentiellement inférieur au dixième, mieux au centième de la distance entre la chambre de confinement 15 et la source de lumière. Sur les figures 1A et 1B, la source de lumière 11 est une diode électroluminescente. Elle est généralement associée à diaphragme 18, ou filtre spatial. L'ouverture du diaphragme est typiquement comprise entre 5 $\mu$m et 1 mm, de préférence entre 50 $\mu$m et 500 $\mu$m. Dans cet exemple, le diaphragme est fourni par Thorlabs sous la référence P150S et son diamètre est de 150 $\mu$m. Le diaphragme peut être remplacé par une fibre optique, dont une première extrémité est placée face à la source de lumière 11 et dont une deuxième extrémité est placée en regard de l'échantillon 10. Le dispositif représenté sur les figures 1A et 1B comporte également un diffuseur 17, disposé entre la source de lumière 11 et le diaphragme 18. L'usage d'un tel diffuseur permet de s'affranchir de contraintes de centrage de la source de lumière 11 par rapport à l'ouverture du diaphragme 18. La fonction d'un tel diffuseur est de répartir le faisceau lumineux, produit par une source de lumière élémentaire 11 selon un cône d'angle $\alpha$. De préférence, l'angle de diffusion $\alpha$ varie entre 10° et 80°. Le recours à un diffuseur a été décrit dans WO2016078946. Alternativement, la source de lumière peut être une source laser, telle une diode laser. Dans ce cas, il n'est pas utile de lui associer un filtre spatial ou un diffuseur.

De préférence, la bande spectrale d'émission $\Delta\lambda$ de l'onde lumineuse incidente 12 a une largeur inférieure à 100 nm. Par largeur de bande spectrale, on entend une largeur à mi-hauteur de ladite bande spectrale.

L'échantillon 10 est disposé entre la source de lumière 11 et le capteur d'image 16 précédemment évoqué. Ce dernier s'étend de préférence parallèlement, ou sensiblement parallèlement à un plan selon lequel s'étend l'échantillon. Le terme sensiblement parallèlement signifie que les deux éléments peuvent ne pas être rigoureusement parallèles, une tolérance angulaire de quelques degrés, inférieure à 20° ou 10° étant admise. Dans cet exemple, l'échantillon s'étend selon un plan XY, perpendiculaire à l'axe de propagation Z.

L'échantillon s'étend entre une face supérieure $10_{sup}$, située en regard de la source de lumière 11, et une face inférieure $10_{inf}$, située en regard du capteur d'image 16. Les faces supérieure et inférieure s'étendent de préférence perpendiculairement ou sensiblement perpendiculairement à l'axe de propagation Z de l'onde lumineuse incidente 12. Par sensiblement perpendiculairement, on entend perpendiculaire en admettant une tolérance angulaire de $\pm20°$ ou $\pm10°$.

Le capteur d'image 16 est apte à former une image I de l'échantillon 10 selon un plan de détection P. Dans l'exemple représenté, il s'agit d'un capteur d'image comportant une matrice de pixels, de type CCD ou un CMOS. Le plan de détection P s'étend de préférence perpendiculairement à l'axe de propagation Z de l'onde lumineuse incidente 12.

Le capteur d'image 16 est relié à une unité de traitement 20, configurée pour traiter les images obtenues par le capteur d'image. L'unité de traitement 20 est reliée à une mémoire 22, configurée pour permettre la réalisation d'opérations de traitement d'image. Dans cet exemple, l'unité de traitement 20 est également reliée à un écran 24.

Dans la configuration représentée sur les figures 1A et 1B, on remarque l'absence d'optique de grossissement ou de formation d'image entre le capteur d'image 16 et l'échantillon 10. Cela n'empêche pas la présence éventuelle de microlentilles de focalisation au niveau de chaque pixel du capteur d'image 16, ces dernières n'ayant pas de fonction de grandissement de l'image acquise par le capteur d'image, leur fonction étant d'optimiser l'efficacité de détection. Dans une telle configuration, dite d'imagerie sans lentille, la distance d entre l'échantillon 10 et la matrice de pixels du capteur d'image 16 est préférentiellement comprise entre 50 $\mu$m et 2 cm, de préférence comprise entre 100 $\mu$m et 2 mm.

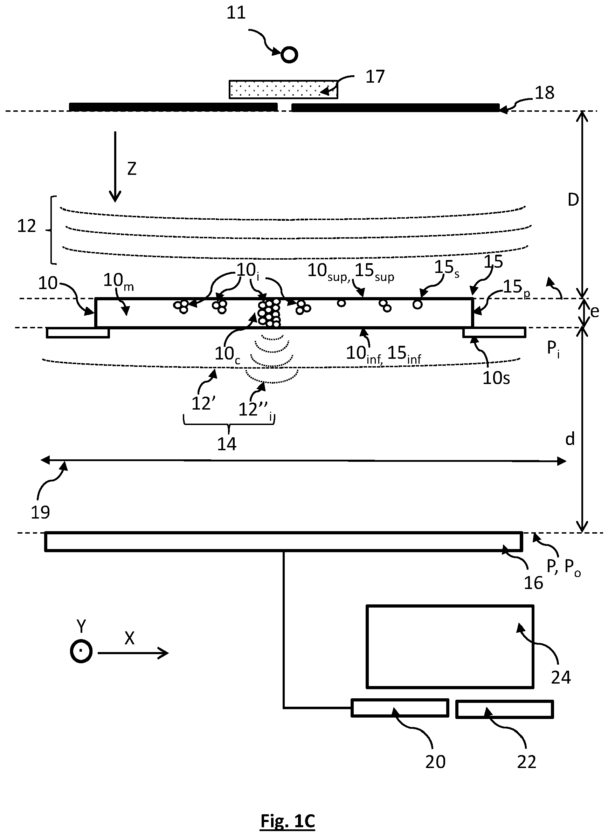

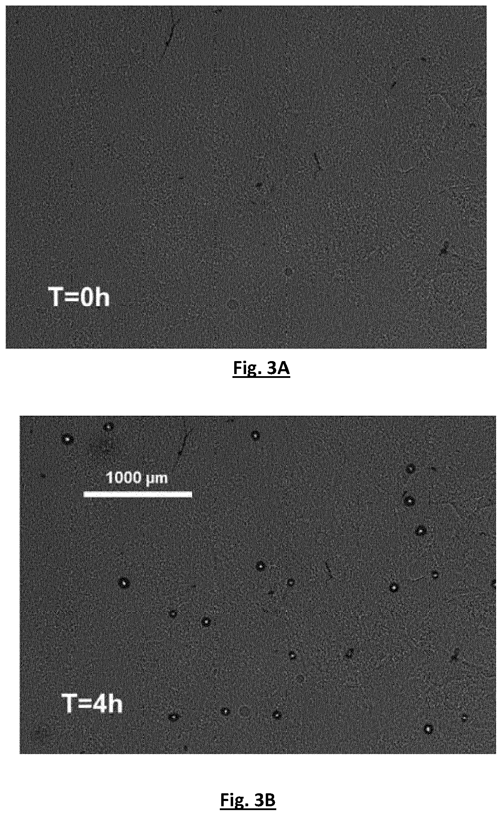

Dans une autre configuration, un système optique 19 de formation d'image peut être disposé entre l'échantillon et le capteur d'image, comme représenté sur la figure 1C. Selon une telle configuration, le système optique peut être un objectif est une lentille. Le système optique 19 définit un plan objet $P_o$ et un plan image $P_i$. Le plan objet se situe de préférence dans l'échantillon, et en particulier au niveau de la face inférieure $10_{inf}$ de l'échantillon. Le plan image $P_i$ est de préférence confondu avec le plan de détection P. De façon à permettre l'observation d'une grande surface de l'échantillon, le grandissement conféré par le système optique 19 est de préférence égal à 1, ou inférieur à 1. Lorsqu'au contraire, on privilégie l'observation de détails, de l'échantillon, le grandissement du système optique 19 peut être supérieur à 1. La configuration représentée sur la figure 1C est une configuration focalisée, le système optique conjuguant l'échantillon, et de façon préférentielle la face inférieure $10_i$, au capteur d'image 16. L'intérêt de la configuration focalisée est expliqué en lien avec la figure 3B.

Selon une autre configuration, dite défocalisée :

- le plan objet $P_o$ est défocalisé par rapport à l'échantillon, d'une distance de défocalisation objet ;
- et/ou le plan image $P_i$ est défocalisé par rapport au plan de détection formé par le capteur d'image, d'une distance de défocalisation image.

La distance de défocalisation image et/ou la distance de défocalisation objet sont de préférence inférieures à 1 mm, voire à 500 $\mu$m.

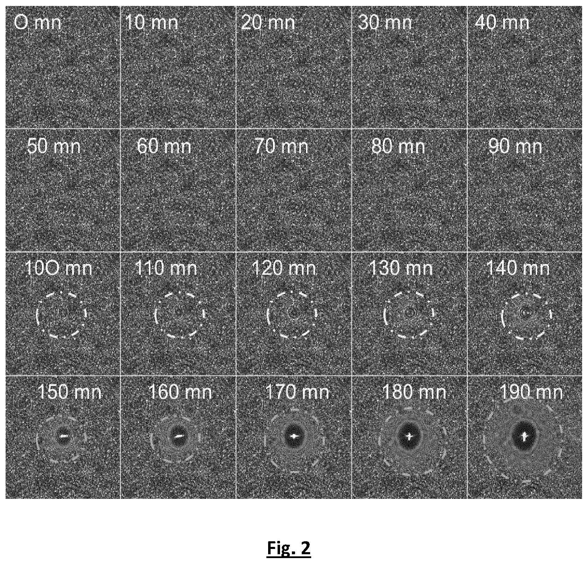

L'intérêt d'une configuration défocalisée est expliqué en lien avec la figure 2.

Le milieu de culture est un milieu liquide ou d'une gélose couramment utilisé en microbiologie. Par exemple, et à titre non limitatif, le milieu de culture peut être :

- un milieu de Chapman, propice au développement germes halophiles et halotolérants, par exemple de, ou bactéries de types *Staphylococcus,* ou *Micrococcus,* ou *Enterococcus,* ou *Bacillus ;*
- un milieu Hektoen, propice au développement de salmonelles ou de shigelles ;
- une gélose Salmonella-Shigella, propice à l'isolement d'entérobactéries pathogènes ;
- un milieu Eosine Bleu de Méthylène (EMB), propice au développement de bactéries Gram négatives ;
- un milieu Mac Conkey, propice au développement de bacilles Gram négatifs, de bactéries coliformes, ou de Salmonella Shigella ;
- un milieu CLED (Cystine Lactose Electrolyte Déficient), couramment utilisé dans l'étude de bactéries contenues dans l'urine, tant Gram + que Gram - ;
- un milieu BCP (Bromocresol Purple), couramment utilisé pour la détection et l'isolement d'entérobactéries ;
- un milieu Baird Parker, propice à l'identification de bactéries de type *Staphylococcus aureus;*
- un milieu BEA (Bile Esculin Agar), propice à l'identification de bactéries de type *Steptococcus ;*
- une gélose au sang (columbia), propice au développement de bactéries de type *Steptococcus ;*
- une gélose au sang cuit (chocolat) ;
- une gélose au kaolin ;
- une gélose de Slantez, usuel pour l'identification de bactéries de type *Enterococcus ;*
- une gélose au cétrimide, permettant l'isolement de bactéries de type *Pseudomonas ;*
- une gélose Sabouraud, pour l'isolement et l'identification de levures ou moisissures saprophytes ou pathogènes ;
- une gélose CIN (Celsulodine Irgasan Novobiocine), permettant l'isolement de bactéries de type *Yersinia enterocolitica ;*
- une gélose au lait ;
- une gélose à l'amidon ;
- une gélose à l'œuf ;
- une gélose Mossel ;
- une gélose Drigalski ;
- une gélose TSN (Tryptone Sulfite Neomycin) ;
- une gélose TCBS (Thiosulfate-citrate-bile salt-sucrose) ;
- une gélose lactosée au désoxycholate ;
- un milieu Muller Hinton ;
- une gélose nutritive ordinaire (Trypcase Soy Agar par exemple) ;
- un milieu Todd Hewit ;
- un milieu TTC tergitol ;
- un milieu Hajna-Kligler ;
- un milieu Lysine Fer ;
- un milieu viande-foie ;
- un milieu de Falkow ;
- un milieu de Môller ;
- un milieu King A ou un milieu King B ;
- un milieu Rappaport ;
- une gélose Esculine agar ;

- une gélose Lowenstein-Jensen ;
- un bouillon BLBVB (bouillon lactosé bilié eu vert brillant). cette liste n'étant pas exhaustive.

Les microorganismes peuvent être ensemencés dans le milieu de culture en profondeur ou en surface.

La plupart des milieux utilisés en microbiologie clinique sont non transparents. Lorsqu'ils sont utilisés dans des boîtes de Pétri classiques, leur épaisseur est de l'ordre de 5 mm, ce qui les rend opaques. Du fait de leur opacité, comme indiqué en lien avec l'art antérieur, il n'est pas envisageable d'acquérir une image exploitable d'un échantillon selon les configurations représentées sur les figures 1A à 1C. Aussi, un élément important de l'invention est que l'épaisseur e de l'échantillon, définie parallèlement à l'axe de propagation Z, est bien plus faible que dans les procédés de l'art antérieur. L'épaisseur de l'échantillon correspond à l'épaisseur du milieu de culture $10_m$. Elle est inférieure à 500 μm, et encore de préférence inférieure à 250 μm, voire à 100 μm. L'épaisseur e est généralement supérieure à 10 ou 20 μm. Selon une telle épaisseur, le milieu de culture $10_m$ devient translucide. Par translucide, on entend qu'il permet une propagation de la lumière à travers son épaisseur, sans être transparent. Par rapport à l'art antérieur, la réduction de l'épaisseur e, permet de former une image de l'échantillon selon une configuration de transmission, l'échantillon s'étendant entre la source de lumière 11 et le capteur d'image 16.

Il est à noter que la réduction de l'épaisseur du milieu de culture $10_m$ est loin d'être évidente. En effet, les méthodes d'observation et de comptage de l'art antérieur supposent que les colonies aient acquis un certain niveau de développement, ce qui suppose une certaine durée entre l'ensemencement du milieu de culture et l'observation des colonies bactériennes. Cette durée est généralement supérieure à 1 jour, voire à plusieurs jours. Cela justifie le recours à un milieu de culture suffisamment épais, de façon à contenir une quantité de nutriments nécessaires à la prolifération des microorganismes. En réduisant l'épaisseur du milieu de culture, les méthodes de l'art antérieur ne seraient pas utilisables, la quantité de nutriments stockée dans le milieu de culture ne permettant pas une durée de culture suffisante.

Les inventeurs ont montré qu'en réduisant l'épaisseur du milieu de culture, des images en transmission peuvent être formées. Ces images sont exploitables et permettent une observation des colonies à un stade de développement précoce. De ce fait, la durée de culture peut être limitée à quelques heures, ce qui rend inutile l'utilisation d'un milieu de culture épais. La quantité de nutriment nécessaire au développement des colonies, avant leur caractérisation, est ainsi considérablement réduite par rapport aux techniques de l'art antérieur. Des exemples d'observation de colonies sont présentés en lien avec les figures 2, 3A et 3B.

L'échantillon 10 est, dans cet exemple, contenu dans une chambre de confinement 15. La chambre de confinement 15 est de préférence transparente. Elle comporte une paroi supérieure $15_{sup}$, une paroi inférieure $15_{inf}$ et une paroi périphérique $15_p$. La paroi périphérique $15_p$ s'étend entre la paroi supérieure $15_{sup}$ et la paroi inférieure $15_{inf}$. De préférence, la paroi périphérique est une paroi annulaire s'étendant autour de l'axe de propagation Z. Les parois inférieures $15_{inf}$ et supérieure $15_{sup}$ sont de préférence perpendiculaires, ou sensiblement perpendiculaires, à l'axe de propagation Z. Les parois supérieures $15_{sup}$ et inférieure $15_{inf}$ sont de préférence disposées au contact des faces supérieure $10_{sup}$ et inférieure $10_{inf}$ de l'échantillon 10, respectivement. Le milieu de culture $10_m$ peut comporter une certaine teneur en oxygène dissous, ce qui permet, dans une certaine mesure, un développement par voie aérobie.

Le confinement de l'échantillon par les parois inférieure et supérieure permet de maintenir une isolation de l'échantillon relativement à l'environnement, ce qui limite les risques de contamination de l'environnement ou de l'échantillon.

La figure 2 montre des images acquises par le capteur d'image en utilisant un dispositif selon une configuration d'imagerie sans lentille, telle que représentée sur les figures 1A et 1B. On a utilisé un échantillon comportant un milieu de culture $10_m$ de type gélose au sang de cheval, obtenue en mélangeant du sang de cheval défibriné à une gélose TSA fondue à 55°C (gélose trypticase soja fournisseur VWR), la fraction volumique de sang ajouté étant de 5%, soit 3 mL pour 60 mL de gélose TSA. On a ensemencé la gélose avec une solution de 600μL contenant des bactéries de type *Escherichia Coli* selon une concentration de 950 CFU (Colony Forming Unit)/mL. L'ensemencement a eu lieu alors que la gélose était toujours liquide. La gélose ensemencée a été coulée dans une chambre Geneframe d'épaisseur variable entre 250 μm et 500 μm.

Les autres paramètres expérimentaux étaient les suivants :

- capteur d'image : CMOS monochrome - taille des pixels 1.67 μm - surface active 30 mm² ;
- source de lumière : LED quatre quadrants CREE MCE-Color ;
- distance source de lumière - capteur d'image : 5 cm ;
- distance échantillon - capteur d'image : 1 mm ;
- diffuseur : Luminit 40° ;
- diaphragme : Thorlabs 150 μm ;

La source de lumière comporte des quadrants émettant respectivement dans une bande spectrale d'illumination rouge (635 nm), verte (520 nm) et bleu (435 nm). Les bandes spectrales d'illumination ont été utilisées séparément.

L'échantillon a été illuminé à l'aide de la source de lumière, dans la bande spectrale 520 nm. On a acquis, toutes les 10 minutes, une image de l'échantillon à l'aide du capteur d'image. La figure 2 montre une même région d'intérêt de 20 images successivement acquises entre t = 0 min et t = 190 min. L'instant t = 0 min correspondant à un instant initial, considéré comme coïncidant de la coulée de la gélose dans la chambre de confinement 15.

Entre t = 100 mn et t = 140 mn, on observe une formation d'une figure de diffraction au centre de chaque image. La figure de diffraction est difficilement distinguable à t = 100 mn et devient de plus en plus discernable, en particulier à t = 130 min et t = 140 mn. La figure de diffraction est formée par une interférence entre :

- une partie 12' de l'onde lumineuse d'illumination 12 émise par la source de lumière, la partie 12' correspondant à la partie de l'onde lumineuse transmise par l'échantillon, c'est-à-dire non absorbée par ce dernier ;
- une onde de diffraction 13, générée par une colonie bactérienne. L'onde de diffraction est d'autant plus visible que le volume de la colonie bactérienne se développe.

La superposition de la partie transmise 12' de l'onde lumineuse d'illumination, et de l'onde de diffraction 13, forme une onde lumineuse d'exposition 14 se propageant jusqu'au capteur d'image, et permettant la détection de la colonie. Une telle onde lumineuse d'exposition est représentée sur la figure 1A. Une figure de diffraction présente généralement une tache centrale, autour de laquelle s'étendent des anneaux concentriques.

La détection, sur l'image formée par le capteur d'image, de telles figures de diffraction, permet de détecter un développement de colonies bactériennes, et d'effectuer un dénombrement de ces dernières, à un stade de développement particulièrement précoce. En effet, chaque colonie peut être associée à une figure de diffraction. En outre, la morphologie de la figure de diffraction peut permettre une identification de chaque colonie. En effet, la morphologie des figures de diffraction est dépendante du type de microorganisme formant la colonie, comme décrit dans l'art antérieur. Ainsi, la présence de figures de diffraction sur l'image acquise par le capteur d'image permet d'obtenir une information qualitative et quantitative à un stade très précoce de développement, par exemple moins de 2 heures après l'ensemencement.

Les figures de diffraction, telles que précédemment décrites, peuvent également être observées selon une configuration défocalisée, telle que précédemment décrite. La distance de défocalisation est alors de préférence inférieure à 1 mm, ou inférieure à 500 $\mu$m, voire à 200$\mu$m ou 100 $\mu$m.

Lorsque la colonie se développe, un phénomène remarquable se produit, comme on peut le voir sur les vignettes correspondant aux instants compris entre t=150 mn à 190 mn. En effet, on observe la formation de points lumineux intense sur l'image acquise par le capteur d'image. Ces zones ponctuelles intenses sont bien délimitées, et permettent d'effectuer une détection et un comptage particulièrement aisés des colonies. Les inventeurs expliquent la présence des zones ponctuelles intenses par le développement des colonies, comme représenté sur la figure 1B. Du fait que l'épaisseur du milieu de culture soit faible, les colonies bactériennes, en se développant, s'étendent de la face supérieure $10_{sup}$ de l'échantillon jusqu'à la face inférieure $10_{inf}$ de ce dernier. Elles forment alors un canal $10_C$ s'étendant à travers le milieu de culture $10_m$, le canal joignant la face supérieure $10_{sup}$ à la face inférieure $10_{inf}$. Le canal $10_c$ forme un guide de lumière, permettant une propagation aisée d'une partie $12"_i$ de l'onde lumineuse incidente, se propageant localement, au niveau d'une colonie $10_i$, à travers le guide de lumière formé par le canal $10_c$.

Ainsi, après un certain stade de développement des colonies, l'onde lumineuse d'exposition 14, parvenant au capteur d'image 16, comporte :

- une première partie 12' de l'onde lumineuse d'illumination 12, transmise par la gélose formant l'échantillon ;
- une deuxième partie $12"_i$ de l'onde lumineuse d'illumination 12, transmise localement par chaque canal $10_c$, au niveau de colonies $10_i$, et formant des zones ponctuelles intenses sur l'image, ou taches lumineuses.

Il en résulte une formation d'une image particulièrement contrastée, où chaque colonie forme une tache intense sur l'image. Cela permet une détection et un comptage particulièrement aisés des colonies formées dans l'échantillon.

Les figures 3A et 3B montrent des exemples d'images de l'échantillon précédemment décrit, formées respectivement à t = 0 min (instant considéré comme correspondant à la coulée de la gélose dans l'échantillon) ainsi qu'à t = 4 h. L'image formée à t = 4h est suffisamment contrastée pour dénombrer les colonies.

Ainsi, la méthode permet :

- à un stade très précoce, la formation de figures de diffraction, témoignant de la présence de colonies bactérienne dans la gélose, et permettant un premier comptage, voire une identification des microorganismes formant chaque colonie. En comparant deux images acquises à deux instants différents, séparés d'un intervalle d'au moins ½ temps de génération, on peut distinguer les figures de diffraction provenant de microcolonies en croissance de figures de diffraction issues d'objets stériles non biologiques que l'on peut trouver classiquement dans des milieux gélosés (poussières, précipités).
- à un stade précoce, c'est-à-dire en quelques heures, typiquement en moins de 6h, la formation d'une image contrastée, formée de taches ponctuelles lumineuses, chaque tache ponctuelle lumineuse correspondant à une colonie. Cela permet un dénombrement aisé et fiable des colonies. Les inventeurs estiment que la forme des taches peut dépendre du type de microorganisme. Ainsi, une analyse morphologique de l'image peut permettre d'obtenir une information qualitative, relative au type de microorganisme se développant dans le milieu de culture.

Les figures 3A et 3B ont été acquises selon une configuration sans lentille, telle que décrite sur les figures 1A et 1B. Des images similaires, voire plus contrastées et plus résolues, peuvent être obtenues en disposant un système optique 19 tel que décrit dans la figure 1C, selon une configuration focalisée. Dans ce cas, le plan objet $P_o$ du système optique 19 est confondu avec la face inférieure $10_{inf}$ de l'échantillon, tandis que le plan image $P_i$ du système optique 19 est confondu avec le plan de détection P.

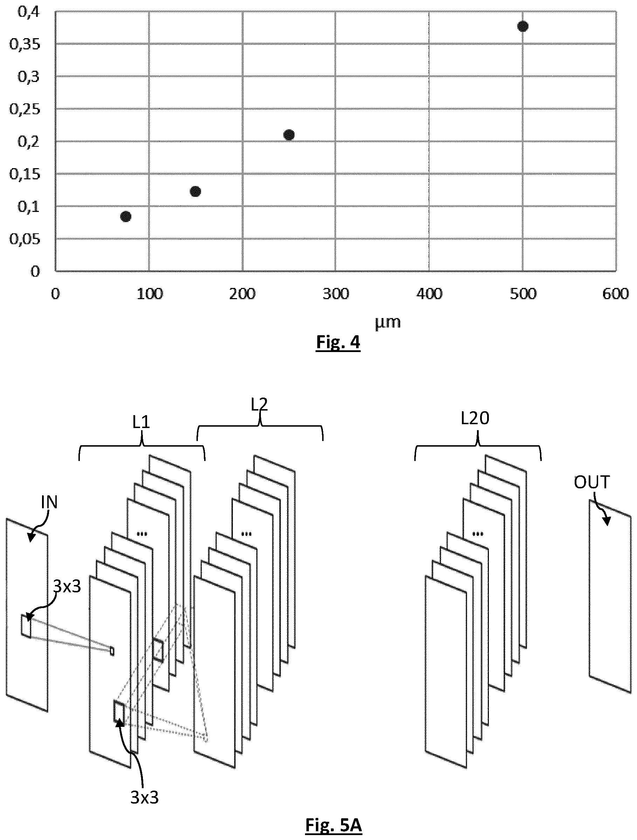

La figure 4 représente une évolution de l'atténuation lumineuse de la gélose au sang précédemment décrite, en fonction de l'épaisseur. Pour obtenir cette figure, on a utilisé une gélose au sang non ensemencée, et dont l'épaisseur a varié entre 75 $\mu$m et 500 $\mu$m. On a évalué l'atténuation formée par la gélose par rapport à une mesure d'une l'intensité lumineuse $I_0$ sans gélose entre la source de lumière et le capteur d'image.

L'atténuation $Att_e$ , correspondant à l'épaisseur e, est obtenue selon l'expression suivante, dérivant de la loi de Beer Lambert :

$$Att_e = -ln\left(\frac{I_e}{I_0}\right) \quad (1)$$

Où $I_e$ est l'intensité lumineuse mesurée en présence d'une épaisseur e de gélose.

Les mesures représentées sur la figure 4 ont été effectuées à la longueur d'onde de 540 nm. Des mesures comparatives ont été effectuées en utilisant une gélose claire, de type TSA. L'atténuation est peu dépendante de l'épaisseur, et reste voisine de 0.05, pour des épaisseurs comprises entre 100 et 500 $\mu$m.

L'invention peut être mise en oeuvre tant que l'atténuation, telle que définie en lien avec l'équation (1), est inférieure à 0.5, et de préférence inférieure à 0.4 voire 0.3.

Les inventeurs estiment que le procédé permet une détection et un dénombrement de colonies dont le diamètre est compris entre 5 $\mu$m et 50 $\mu$m, ou davantage. Le procédé permet une détection particulièrement précoce des colonies.

Selon un mode de réalisation non revendiqué, le dénombrement de figures de diffraction, telles que représentées sur la figure 2, est effectué en mettant en oeuvre un algorithme de reconnaissance automatique, de type réseaux de neurones. Plus précisément, l'algorithme peut mettre en oeuvre un réseau de neurones convolutif. Le réseau de neurones comporte une couche d'entrée IN, formée d'une image acquise par le capteur d'image, et une couche de sortie OUT, correspondant à une image sur laquelle les colonies de microorganismes sont détectées. Entre la couche d'entrée IN et la couche de sortie OUT, le réseau de neurones comporte 20 couches L1, L2...L20, dont les rangs sont compris entre 1 (couche adjacente de la couche IN) à 20 (couche adjacente de la couche OUT). Chaque couche comporte 32 plans. Une couche est obtenue par une convolution des 32 plans de la couche de rang précédent par un noyau de convolution de taille 3 $\times$ 3. La couche IN est considérée comme la couche de rang 0. La figure 5A schématise une architecture d'un tel réseau.

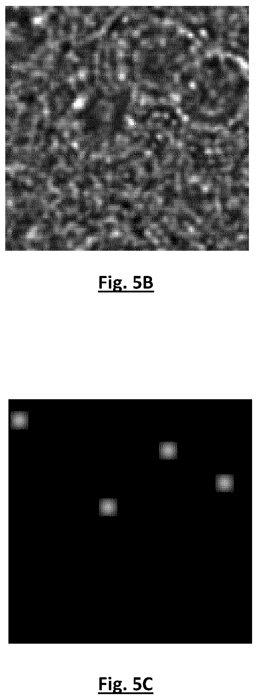

Le réseau de neurones convolutif a fait l'objet d'un apprentissage, en considérant 1000 vignettes d'entrée, chaque vignette d'entrée étant de taille 121 $\times$ 121 pixels. Les vignettes d'entrée ont été prises aléatoirement sur une image d'hologramme. Une telle vignette d'entrée est représentée sur la figure 5B. Pour chaque vignette d'entrée, une vignette de sortie a été déterminée par un expert. La vignette de sortie montre la localisation des microorganismes. Un exemple de vignette de sortie est représenté sur la figure 5C. L'apprentissage a permis de paramétrer le réseau de neurones.

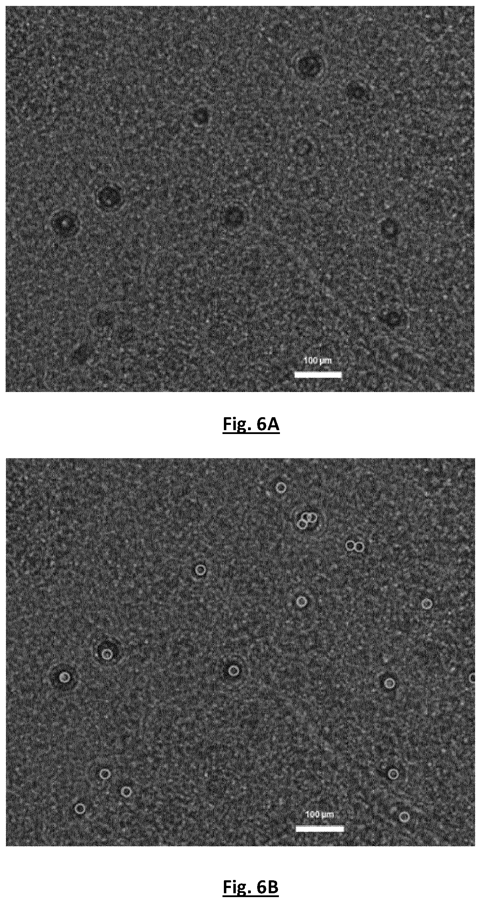

Suite à l'apprentissage, le réseau de neurones a été mis en oeuvre pour détecter automatiquement des colonies de microorganismes. La figure 6A est un exemple d'image acquise par le capteur d'image. Chaque figure de diffraction correspond à des colonies de bactéries de type *Escherichia coli.* La figure 6B montre une détection automatique des colonies, chaque détection colonie détectée étant matérialisée par un cercle clair. Ainsi, l'utilisation d'un réseau de neurones permet une détection, et un dénombrement, ainsi qu'une localisation, de colonies, et cela à un stade de développement précoce.

Par ailleurs, en réalisant des apprentissages sur différentes espèces de microorganismes, le recours à un réseau de neurones peut permettre une détection, un comptage ainsi qu'une identification des microorganismes.

L'invention pourra être utilisée pour réaliser des contrôles et la numération de bactéries, à des fins d'aide au diagnostic médical, ou dans le domaine du contrôle de l'environnement, ou encore du contrôle de procédés industriels, par exemple dans le domaine de l'agroalimentaire ou des cosmétiques.

**Revendications**

1. Procédé d'observation d'un échantillon (10), l'échantillon comportant des colonies de microorganismes ($10_i$) baignant dans un milieu de culture ($10_m$) non transparent, le milieu de culture étant propice au développement des microorganismes, l'échantillon étant disposé entre une source de lumière (11) et un capteur d'image (16), le procédé comportant:

a) illumination de l'échantillon par la source de lumière (11), la source de lumière émettant une onde lumineuse incidente se propageant selon un axe de propagation (Z);
b) acquisition d'une image (I) de l'échantillon par le capteur d'image (16) à un instant initial et à un instant de mesure;
c) à partir de l'image acquise, détection des microorganismes ;

le procédé étant **caractérisé en ce que** :

- le milieu de culture ($10_m$) s'étend, parallèlement à l'axe de propagation, selon une épaisseur inférieure à 500 $\mu$m.
- le milieu de culture s'étend entre une face supérieure ($10_{sup}$) et une face inférieure ($10_{inf}$), perpendiculaires ou perpendiculaires à 20° près, à l'axe de propagation;
- sous l'effet de leur développement, les microorganismes forment des colonies, au moins une colonie formant un canal de lumière ($10_c$) s'étendant de la face supérieure ($10_{sup}$) jusqu'à la face inférieure ($10_{inf}$) à travers le milieu de culture;

de telle sorte :

- qu'au moins une colonie forme une tache lumineuse, apparaissant sur l'image acquise par le capteur d'image entre l'instant initial et l'instant de mesure, chaque tache lumineuse correspondant à une zone ponctuelle intense ;
- que l'étape c) comporte une détection des taches lumineuses sur l'image acquise par le capteur d'image à l'instant de mesure.

2. Procédé selon la revendication 1, dans lequel le milieu de culture ($10_m$) s'étend, parallèlement à l'axe de propagation, selon une épaisseur inférieure à 250 $\mu$m ou à 100 $\mu$m.

3. Procédé selon l'une quelconque des revendications précédentes, comportant :

- un comptage de taches lumineuses formées sur l'image acquise par le capteur d'image;
- une estimation du nombre de colonies dans l'échantillon, en fonction du nombre de taches lumineuses comptées sur l'image.

4. Procédé selon l'une quelconque des revendications précédentes comportant :

- une analyse morphologique d'au moins une tache lumineuse formée sur l'image acquise par le capteur d'image;
- une identification de la colonie, ayant engendré la tache lumineuse, à partir de l'analyse morphologique.

5. Procédé selon l'une quelconque des revendications précédentes, dans lequel :

- le milieu de culture s'étend dans une chambre de confinement (15);
- le milieu de culture s'étend entre deux faces opposées ($15_{sup}$, $15_{inf}$), perpendiculaires ou sensiblement perpendiculaires à l'axe de propagation ;
- la chambre de confinement est au contact du milieu de culture au niveau des deux faces opposées.

6. Procédé selon l'une quelconque des revendications précédentes, dans lequel aucune optique de formation d'image n'est disposée entre l'échantillon et le capteur d'image.

7. Procédé selon l'une quelconque des revendications 1 à 5, dans lequel :

- un système optique (19) de formation d'image est disposé entre l'échantillon et le capteur d'image, le système optique définissant un plan objet ($P_o$) et un plan image ($P_i$) ;
- le capteur d'image définit un plan de détection (P) ;
le procédé étant tel que, lors de l'acquisition de l'image :

- l'échantillon est décalé par rapport au plan objet selon une distance de défocalisation objet ;
- et/ou le plan de détection est décalé, par rapport au plan image, selon une distance de défocalisation image.

8. Procédé selon l'une quelconque des revendications précédentes, dans lequel

- un système optique de formation d'image (19) est disposé entre l'échantillon et le capteur d'image, le système optique définissant un plan objet ($P_o$) et un plan image ($P_i$) ;
- le capteur d'image définit un plan de détection (P) ;
le procédé étant tel que, lors de l'acquisition de l'image, la face inférieure de l'échantillon correspond au plan objet et le plan de détection correspond au plan image.

**Patentansprüche**

1. Verfahren zur Beobachtung einer Probe (10), wobei die Probe Mikroorganismen-Kolonien (10i) aufweist, die in einem nicht transparenten Kulturmedium (10m) schwimmen, wobei das Kulturmedium für die Entwicklung der Mikroorganismen geeignet ist, wobei die Probe zwischen einer Lichtquelle (11) und einem Bildsensor (16) angeordnet ist, das Verfahren aufweisend:

   a) Beleuchten der Probe durch die Lichtquelle (11), wobei die Lichtquelle eine einfallende Lichtwelle emittiert, die sich gemäß einer Ausbreitungsachse (Z) ausbreitet;
   b) Erfassen eines Bilds (I) der Probe durch den Bildsensor (16) zu einem Anfangszeitpunkt und einem Messzeitpunkt;
   c) Detektieren der Mikroorganismen anhand des erfassten Bilds;
   wobei das Verfahren **dadurch gekennzeichnet ist, dass**:

   - sich das Kulturmedium (10m) parallel zur Ausbreitungsachse gemäß einer Dicke unter 500 $\mu$m erstreckt.
   - sich das Kulturmedium zwischen einer Oberseite (10sup) und einer Unterseite (10inf) erstreckt, die senkrecht oder bis auf 20° genau senkrecht zur Ausbreitungsachse verlaufen;
   - die Mikroorganismen unter der Wirkung ihrer Entwicklung Kolonien bilden, wobei wenigstens eine Kolonie einen Lichtkanal (10c) bildet, der sich von der Oberseite (10sup) bis zur Unterseite (10inf) durch das Kulturmedium erstreckt;
   so dass:

   - wenigstens eine Kolonie einen Lichtfleck bildet, der auf dem Bild, das vom Bildsensor erfasst wird, zwischen dem Anfangszeitpunkt und dem Messzeitpunkt erscheint, wobei jeder Lichtfleck einem intensiven punktuellen Bereich entspricht;
   - der Schritt c) ein Detektieren der Lichtflecken auf dem Bild umfasst, das vom Bildsensor zum Messzeitpunkt erfasst wird.

2. Verfahren nach Anspruch 1, wobei sich das Kulturmedium (10m) parallel zur Ausbreitungsachse gemäß einer Dicke unter 250 $\mu$m oder 100 $\mu$m erstreckt.

3. Verfahren nach einem der vorhergehenden Ansprüche, aufweisend:

   - ein Zählen von Lichtflecken, die auf dem Bild gebildet sind, das vom Bildsensor erfasst wird;
   - ein Schätzen der Anzahl von Kolonien in der Probe in Abhängigkeit von der Anzahl von Lichtflecken, die auf dem Bild gezählt werden.

4. Verfahren nach einem der vorhergehenden Ansprüche, aufweisend:

   - eine morphologische Analyse wenigstens eines Lichtflecks, der auf dem Bild gebildet ist, das vom Bildsensor erfasst wird;
   - ein Identifizieren der Kolonie, die den Lichtfleck herbeigeführt hat, anhand der morphologischen Analyse.

5. Verfahren nach einem der vorhergehenden Ansprüche, wobei:

   - sich das Kulturmedium in einer Isolierkammer (15) erstreckt;
   - sich das Kulturmedium zwischen zwei gegenüberliegenden Seiten (15sup, 15inf) erstreckt, die senkrecht oder im Wesentlichen senkrecht zur Ausbreitungsachse verlaufen;
   - sich die Isolierkammer an den zwei gegenüberliegenden Seiten in Kontakt mit dem Kulturmedium befindet.

6. Verfahren nach einem der vorhergehenden Ansprüche, wobei keine Bilderzeugungsoptik zwischen der Probe und dem Bildsensor angeordnet ist.

7. Verfahren nach einem der Ansprüche 1 bis 5, wobei:

   - ein optisches System (19) zur Bilderzeugung zwischen der Probe und dem Bildsensor angeordnet ist, wobei das optische System eine Objektebene (Po) und eine Bildebene (Pi) definiert;
   - der Bildsensor eine Detektionsebene (P) definiert;
   wobei das Verfahren so geartet ist, dass bei der Erfassung des Bilds:

   - die Probe um einen Objekt-Defokussierungsabstand bezogen auf die Objektebene versetzt ist;
   - und/oder die Detektionsebene um einen Bild-Defokussierungsabstand bezogen auf die Bildebene versetzt ist.

8. Verfahren nach einem der vorhergehenden Ansprüche, wobei

   - ein optisches System zur Bilderzeugung (19) zwischen der Probe und dem Bildsensor angeordnet ist, wobei das optische System eine Objektebene (P0) und eine Bildebene (Pi) definiert;

- der Bildsensor eine Detektionsebene (P) definiert;

wobei das Verfahren so geartet ist, dass bei der Erfassung des Bilds die Unterseite der Probe der Objektebene und die Detektionsebene der Bildebene entspricht.

## Claims

1. Method for observing a sample (10), the sample comprising colonies of microorganisms (10i) bathing in a non-transparent culture medium ($10_m$), the culture medium favouring the development of the microorganisms, the sample being disposed between a light source (11) and an image sensor (16), the method comprising:

   a) illumination of the sample by the light source (11), the light source emitting an incident light wave being propagated along an axis of propagation (Z);
   b) acquisition of an image (I) of the sample by the image sensor (16) at an initial instant and at a measurement instant;
   c) from the acquired image, detection of the microorganisms;

   the method being **characterized in that**:

   - the culture medium ($10_m$) extends, parallel to the axis of propagation, to a thickness less than 500 $\mu$m;
   - the culture medium extends between a top face ($10_{sup}$) and a bottom face ($10_{inf}$), at right angles, or at right angles to within 20°, to the axis of propagation;
   - under the effect of their development, the microorganisms form colonies, at least one colony forming a light channel ($10_c$) extending from the top face ($10_{sup}$) to the bottom face ($10_{inf}$) through the culture medium;

   in such a way:

   - that at least one colony forms a light spot, appearing on the image acquired by the image sensor between the initial instant and the measurement instant, each light spot corresponding to an intense isolated zone;
   - that the step c) comprises a detection of the light spots on the image acquired by the image sensor at the measurement instant.

2. Method according to Claim 1, wherein the culture medium ($10_m$) extends, parallel to the axis of propagation, to a thickness less than 250 $\mu$m or 100 $\mu$m.

3. Method according to either one of the preceding claims, comprising:

   - a counting of light spots formed on the image acquired by the image sensor;
   - an estimation of the number of colonies in the sample, as a function of the number of light spots counted on the image.

4. Method according to any one of the preceding claims, comprising:

   - a morphological analysis of at least one light spot formed on the image acquired by the image sensor;
   - an identification of the colony, having generated the light spot, from the morphological analysis.

5. Method according to any one of the preceding claims, wherein:

   - the culture medium extends in a containment chamber (15);
   - the culture medium extends between two opposite faces ($15_{sup}$, $15_{inf}$), at right angles or substantially at right angles to the axis of propagation;
   - the containment chamber is in contact with the culture medium on the two opposite faces.

6. Method according to any one of the preceding claims, wherein no image forming optic is disposed between the sample and the image sensor.

7. Method according to any one of Claims 1 to 5, wherein

   - an image forming optical system (19) is disposed between the sample and the image sensor, the optical system defining an object plane ($P_o$) and an image plane ($P_i$);
   - the image sensor defines a detection plane (P);
   the method being such that, upon the acquisition of the image:

   - the sample is offset with respect to the object plane by an object defocussing distance;
   - and/or the detection plane is offset, with respect to the image plane, by an image defocussing distance.

8. Method according to any one of the preceding claims, wherein

- an image forming optical system (19) is disposed between the sample and the image sensor, the optical system defining an object plane ($P_o$) and an image plane ($P_i$);
- the image sensor defines a detection plane (P); the method being such that, upon the acquisition of the image, the bottom face of the sample corresponds to the object plane and the detection plane corresponds to the image plane.

- an image forming optical system (19) is disposed between the sample and the image sensor, the optical system defining an object plane ($P_o$) and an image plane ($P_i$);

**Fig. 1A**

**Fig. 1B**

**Fig. 1C**

**Fig. 2**

**Fig. 3A**

**Fig. 3B**

**Fig. 4**

**Fig. 5A**

**Fig. 5B**

**Fig. 5C**

**Fig. 6A**

**Fig. 6B**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 7465560 B
- US 8787633 B
- WO 2018122504 A
- WO 2018122505 A
- WO 2018215337 A
- FR 3054037
- WO 2016078946 A

**Littérature non-brevet citée dans la description**

- **MEADA Y.** Colony fingerprint for discrimination of microbial species based on lensless imaging of microcolonies. *PLoS ONE,* 2017, vol. 12 (4